Europäisches Patentamt

European Patent Office

Office européen des brevets

(19)

(11) Publication number: **0 085 533**
**A2**

# (12) EUROPEAN PATENT APPLICATION

(21) Application number: 83300385.8

(22) Date of filing: 26.01.83

(51) Int. Cl.³: **A 61 F 11/02**

(30) Priority: 28.01.82 GB 8202379

(43) Date of publication of application:
10.08.83 Bulletin 83/32

(84) Designated Contracting States:
BE DE FR NL SE

(71) Applicant: CHAPMAN & SMITH LIMITED
Safir Works East Hoathly
Nr Lewes East Sussex BN8 6EW(GB)

(72) Inventor: Connor, Dennis Frederick
11 Southcourt Avenue
Bexhill-on-Sea East Sussex(GB)

(72) Inventor: Smith, Maurice Arthur
Little Bourton Stepham Lane
Polegate East Sussex BN26 6NB(GB)

(74) Representative: Spence, Anne et al,
Barlin Professional Services Barlin House 20 High Street
Carshalton, Surrey SM5 3AG(GB)

(54) Ear defender and diaphragm therefor.

(57) An Ear Defender has a cup shaped body (12) in which a cup shaped diaphragm (13) of poor sound conducting material, such as rubber, is located to provide a sound insulating chamber. The lip (16) of the diaphragm is fixed to the rim of the body (12) by annular clamping plate (14) which also provides a retaining edge for a loosly located foamed pad (21).

FIG. 2

EP 0 085 533 A2

Croydon Printing Company Ltd

EAR DEFENDER AND DIAPHRAGM THEREFOR

This invention relates to Ear Defenders and diaphragms
therefor.

Commonly an Ear Defender comprises a cup shaped body
filled with foam material to provide sound insulation.
The rigid plastics material from which the body is made
conducts sound through it.

An object of the present invention is to provide an
Ear Defender having improved sound insulation.

Accordingly the invention provides an Ear Defender
comprising a cup shaped body and a flexible diaphragm
mounted on the body to form a sound insulation air chamber
within the body.

The invention extends to a diaphragm for mounting
on the cup shaped body of an Ear Defender.

The diaphragm is preferably made of material which
is a poor conductor of sound, preferably of rubber,
flexible plastics such as polyurethane or coated cloth
material, for example, woven cotton dipped in polyvinylchloride.

The rim or lip of the diaphragm may be held between
the rim of the body and an annular clamping plate secured
thereto to produce a semi-sealed air chamber.

One embodiment of Ear Defender in accordance with the
invention will now be described, by way of example only,
with reference to the accompanying drawings of which:-

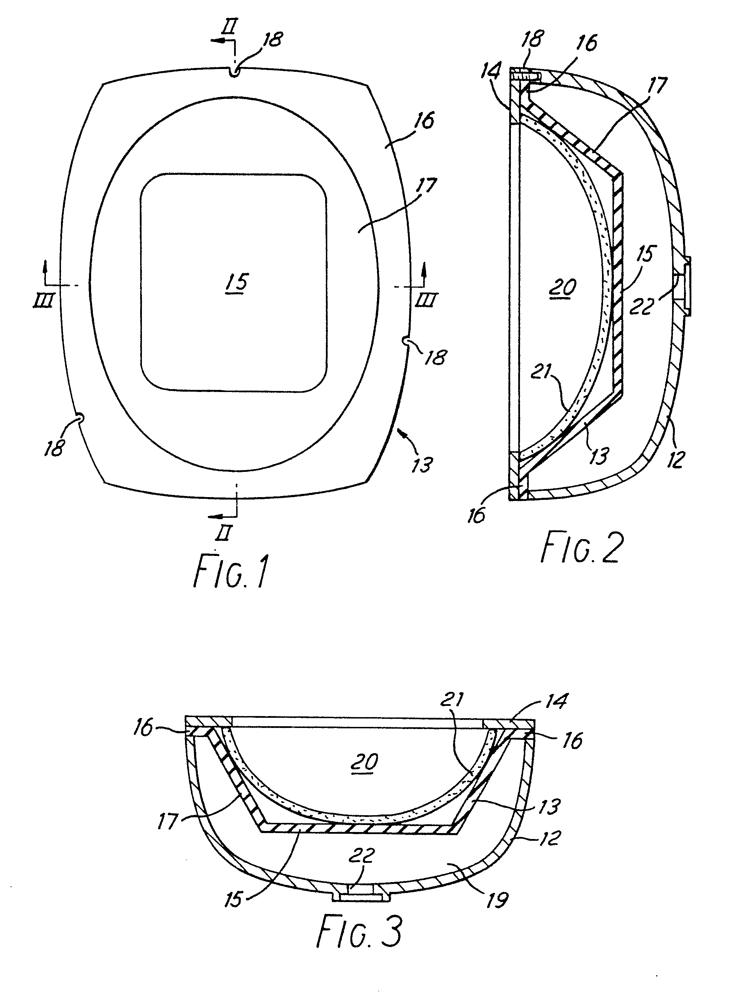

Figure 1 is a plan view of a diaphragm in an Ear
Defender with the clamping plate removed,

Figure 2 is a section on the line II-II of Figure 1 with the clamping plate in position, and

Figure 3 is a section on the line III-III of Figure 1 with the clamping plate in position.

The Ear Defender comprises a rigid plastics cup shaped body 12, a rubber diaphragm 13 and an annular clamping plate 14. The diaphragm is itself of dished or bowl shape having a substantially planar base portion 15 joined to a parallel planar outwardly turned lip portion 16 by sloping walls 17.

The lip 16 of the diaphragm is clamped between the rim of the body and the annular plate 14 which is held to the body by three screws at positions 18. A sound insulating air chamber 19 is formed in the body between the diaphragm and the body. The lip 16 provides sound insulating material between the body and the plate 14 which in use is held against the head surrounding the ear.

The base of the diaphragm extends more than half way into the depth of the body so as to provide adequate space 20 for an ear. The plate 14 defines an oval opening for the ear of smaller dimensions than the oval inner edge of the lip and a foamed plastics material pad 21 is loosely located in the space 20 with its ends tucked under the overhanging inner edge of the plate. This pad readily absorbs dirt such as dust or sweat in use and can be frequently changed.

The diaphragm has a thickness of 2 mm, this thickness preferably being in the range 1.5 mm. to 2.5 mm.

Aperture 22 is for fixing the body to a headband.

CLAIMS

1.    An Ear Defender comprising a cup shaped body adapted to fit around a human ear characterised by a flexible diaphragm (13) mounted in the body to form a sound insulation air chamber (19) within the body.

2.    An Ear Defender according to Claim 1 characterised in that the diaphragm (13) is made of a material which is a poor sound conductor.

3.    An Ear Defender according to Claim 1 or Claim 2 characterised in that the diaphragm (13) is itself dished or bowl shaped.

4.    An Ear Defender according to Claim 3 characterised in that the diaphragm (13) has a substantially planar base portion (15) joined to a substantially planar parallel outwardly turned lip (16) by intermediate walls (17).

5.    An Ear Defender according to Claim 3 or Claim 4 characterised in that the base (15) of the diaphragm (13) extends more than half way into the depth of the body (12).

6.    An Ear Defender according to any of Claims 1 to 5 characterised in that the diaphragm (13) has a lip (16) secured to the rim of the body by a clamping member (14).

7.    An Ear Defender according to Claim 6 characterised in that the clamping member (14) defines an opening for an ear which is smaller than the opening defined by the inner edge of the lip (16) so as to provide an overlapping retaining edge.

8.    An Ear Defender according to Claim 7 characterised by

an attenuating pad loosely located in the diaphragm
and retained by the retaining edge.

9.    A diaphragm for use in an Ear Defender according
to any of the preceeding claims.

1 / 1

0085533

FIG.1

FIG.2

FIG.3